# EUROPEAN PATENT APPLICATION

(11) **EP 1 291 423 A1**
(43) Date of publication of application: **12.03.2003**
(21) Application number: 01938687.9
(22) Date of filing: 15.06.2001
(51) Int. Cl.: C12N 15/11, C12N 1/21, C12N 1/19, C12N 5/10, C12P 21/02, C07K 14/00, A01H 5/00, A01K 67/027, A61K 38/00

(54) **POLYFUNCTIONAL BASE SEQUENCE AND ARTIFICIAL GENE CONTAINING THE SAME**

(30) Priority: 16.06.2000 JP 2000180997
(71) Applicant: JAPAN SCIENCE AND TECHNOLOGY CORPORATION, Kawaguchi-shi, Saitama 332-0012 (JP)
(72) Inventor: SHIBA, Kiyotaka, Toshima-ku, Tokyo 170-0012 (JP)
(74) Representative: HOFFMANN - EITLE
(86) International application number: JP0105116
(87) International publication number: WO01096558

(57) **Abstract**

The present invention provides: a microgene comprising a multifunctional base sequence having two or more functions in different reading frames of the base sequence which is required for creating industrially useful artificial proteins that do not exist in nature; an artificial gene obtained by polymerizing the microgenes; and an artificial protein which is a translation product of the artificial gene.

A microgene is produced by selecting a base sequence by the computational science approach from among all combinations of base sequences encoding an amino acid sequence having a given function where the selected base sequence has a biological function same as or different from the given function in a reading frame different from that of the amino acid sequence having the given function, and is devoid of termination codons in all three reading frames; the microgene thus obtained is then polymerized in such a manner as plural reading frames emerge to produce an artificial gene; and the artificial protein as a translation product of the artificial gene is obtained.

SEQUENCE LISTING

<110> JAPAN SCIENCE AND TECHNOLOGY CORPORATION

<120> Multifunctional base sequence and artificial genes
      which comprise the said sequence

<130> YC13-10PCT

<140>
<141>

<150> JP 2000-180997
<151> 2000-06-16

<160> 34

<170> PatentIn Ver. 2.1

<210> 1
<211> 20
<212> PRT
<213> Artificial Sequence

<220>
<223> Description of Artificial Sequence:Designed
      Peptide

<400> 1
Arg Lys Ser Ile Arg Ile Gln Arg Gly Pro Gly Arg Thr Phe Val Thr
  1               5                  10                  15

Ile Gly Lys Ile
             20


<210> 2
<211> 6
<212> PRT
<213> Artificial Sequence

<220>
<223> Description of Artificial Sequence:Designed
      Peptide

<400> 2

Arg Lys Ser Ile Arg Ile
1               5

<210> 3
<211> 13
<212> PRT
<213> Artificial Sequence

<220>
<223> Description of Artificial Sequence:Designed
      Peptide

<400> 3
Ile Arg Ile Gln Arg Gly Pro Gly Arg Thr Phe Val Thr
1               5                   10

<210> 4
<211> 7
<212> PRT
<213> Artificial Sequence

<220>
<223> Description of Artificial Sequence:Designed
      Peptide

<400> 4
Phe Val Thr Ile Gly Lys Ile
1               5

<210> 5
<211> 13
<212> PRT
<213> Artificial Sequence

<220>
<223> Description of Artificial Sequence:Designed
      Peptide

<400> 5
Tyr Ala Phe Arg Glu Ala Leu Ala Ala Leu Leu Leu Leu
1               5                   10

<210> 6
<211> 39
<212> DNA
<213> Artificial Sequence

<220>
<223> Description of Artificial Sequence:Designed Base
      Sequence

<400> 6
atacgcattc agagaggccc tggccgcact tttgttact                    39


<210> 7
<211> 6
<212> PRT
<213> Artificial Sequence

<220>
<223> Description of Artificial Sequence:Desighed
      Peptide

<400> 7
Glu Arg Ala Tyr Ala Phe
  1               5


<210> 8
<211> 18
<212> DNA
<213> Artificial Sequence

<220>
<223> Description of Artificial Sequence:Designed Base
      Sequence

<400> 8
cgaaagagca tacgcatt                                           18


<210> 9
<211> 7
<212> PRT
<213> Artificial Sequence

<220>

<223> Description of Artificial Sequence:Designed
      Peptide

<400> 9
Leu Leu Leu Leu Glu Arg Tyr
  1               5


<210> 10
<211> 21
<212> DNA
<213> Artificial Sequence

<220>
<223> Description of Artificial Sequence:Designed Base
      Sequence

<400> 10
tttgttacta ttggaaagat a                                                21


<210> 11
<211> 60
<212> DNA
<213> Artificial Sequence

<220>
<223> Description of Artificial Sequence:Designed Base
      Sequence

<400> 11
cgaaagagca tacgcattca gagaggccct ggccgcactt ttgttactat tggaaagata 60


<210> 12
<211> 34
<212> DNA
<213> Artificial Sequence

<220>
<223> Description of Artificial Sequence:Synthesized
      Base Sequence

<400> 12
cgaaagagca tacgcattca gagaggccct ggca                                  34

<210> 13
<211> 36
<212> DNA
<213> Artificial Sequence

<220>
<223> Description of Artificial Sequence:Synthesized
      Base Sequence

<400> 13
tatctttcca atagtaacaa aagtgcggcc agggca                                36


<210> 14
<211> 221
<212> DNA
<213> Artificial Sequence

<220>
<223> Description of Artificial Sequence:Designed Base
      Sequence

<400> 14
cgaaagagca tacgcattca gagaggccct ggccgcactt ttgttactat tggcgaaaga 60
gcatacgcat tcagagaggc cctggccgca cttttgttac tattggacga aagagcatac 120
gcattcagag aggccctggc cgcacttttg ttactattgg aaagatcgaa agagcatacg 180
cattcagaga ggccctggcc gcacttttgt tactattgga g                         221


<210> 15
<211> 323
<212> DNA
<213> Artificial Sequence

<220>
<223> Description of Artificial Sequence:Designed Base
      Sequence

<400> 15
gaaaaagcat acgcattcag agaggccctg gccgcacttt tgttactatt ggaaagatag 60
agcatacgca ttcagagagg ccctggccgc acttttgtta ctattggaaa gatagcatac 120
gcattcagag aggccctggc cgcacttttg ttactattgg cgaaagagca tacgcattca 180
gagaggccct ggccgcactt ttgttactat tgcgaaagag catacgcatt cagagaggcc 240
ctggccgcac ttttgttact attgggcgaa agagcatacg cattcagaga ggccctggcc 300
gcacttttgt tactattgga aag                                             323

<210> 16
<211> 326
<212> DNA
<213> Artificial Sequence

<220>
<223> Description of Artificial Sequence:Designed Base
      Sequence

<400> 16
cgaaagagca tacgcattca gagaggccct ggccgcactt ttgttactat tggaagcgaa 60
agagcatacg cattcagaga ggccctggcc gcacttttgt tactattgga aacgaaagag 120
catacgcatt cagagaggcc ctggccgcac ttttgttact attggcgaaa gagcatacgc 180
attcagagag gccctggccg cacttttgtt actattggaa agacgaaaga gcatacgcat 240
tcagagaggc cctggccgca cttttgttta ctattggaaa gatacgaaag agcatacgca 300
ttcagagagg ccctggccgc actttt                                      326


<210> 17
<211> 327
<212> DNA
<213> Artificial Sequence

<220>
<223> Description of Artificial Sequence:Designed Base
      Sequence

<400> 17
cgaaagacat acgcattcag agaggccctg gccgcacttt tgttactatt ggcgaaagag 60
catacgcatt cagagaggcc ctggccgcac ttttgttact attggcgaaa gagcatacgc 120
attcagagag gccctggccg cacttttgtt actattggaa agcgaaagag catacgcatt 180
cagagaggcc ctggccgcac ttttgttact attggaaagc gaaagagcat acgcattcag 240
agaggccctg gccgcacttt tgttactatt ggcgaaagaa catacgcatt cagagaggcc 300
ctggccgcac ttttgttact attggcg                                     327


<210> 18
<211> 94
<212> PRT
<213> Artificial Sequence

<220>
<223> Description of Artificial Sequence:Designed
      Peptide

<400> 18
Met Arg Gly Ser His His His His His His Gly Ser Val Asp Gly Thr
1               5                   10                  15

Pro Lys Glu His Thr His Ser Glu Arg Pro Trp Pro His Phe Cys Tyr
            20                  25                  30

Tyr Trp Arg Lys Ser Ile Arg Ile Gln Arg Gly Pro Gly Arg Thr Phe
        35                  40                  45

Val Thr Ile Gly Arg Lys Ser Ile Arg Ile Gln Arg Gly Pro Gly Arg
    50                  55                  60

Thr Phe Val Thr Ile Gly Lys Ile Glu Arg Ala Tyr Ala Phe Arg Glu
65                  70                  75                  80

Ala Leu Ala Ala Leu Leu Leu Leu Leu Glu Gly Asp Leu Gly
                85                  90


<210> 19
<211> 128
<212> PRT
<213> Artificial Sequence

<220>
<223> Description of Artificial Sequence:Designed
      Peptide

<400> 19
Met Arg Gly Ser His His His His His His Gly Ser Val Asp Gly Thr
1               5                   10                  15

Arg Lys Ser Ile Arg Ile Gln Arg Gly Pro Gly Arg Thr Phe Val Thr
            20                  25                  30

Ile Gly Lys Ile Glu His Thr His Ser Glu Arg Pro Trp Pro His Phe
        35                  40                  45

Cys Tyr Tyr Trp Lys Asp Ser Ile Arg Ile Gln Arg Gly Pro Gly Arg
    50                  55                  60

Thr Phe Val Thr Ile Gly Glu Arg Ala Tyr Ala Phe Arg Glu Ala Leu
65                  70                  75                  80

Ala Ala Leu Leu Leu Leu Leu Arg Lys Ser Ile Arg Ile Gln Arg Gly
                85                  90                  95

Pro Gly Arg Thr Phe Val Thr Ile Gly Arg Lys Ser Ile Arg Ile Gln
            100                 105                 110

Arg Gly Pro Gly Arg Thr Phe Val Thr Ile Gly Lys Gly Asp Leu Gly
        115                 120                 125

<210> 20
<211> 130
<212> PRT
<213> Artificial Sequence

<220>
<223> Description of Artificial Sequence:Designed
      Peptide

<400> 20
Met Arg Gly Ser His His His His His His Gly Ser Val Asp Gly Thr
  1               5                  10                  15

Pro Lys Glu His Thr His Ser Glu Arg Pro Trp Pro His Phe Cys Tyr
            20                  25                  30

Tyr Trp Lys Arg Lys Ser Ile Arg Ile Gln Arg Gly Pro Gly Arg Thr
        35                  40                  45

Phe Val Thr Ile Gly Asn Glu Arg Ala Tyr Ala Phe Arg Glu Ala Leu
    50                  55                  60

Ala Ala Leu Leu Leu Leu Leu Ala Lys Glu His Thr His Ser Glu Arg
 65                  70                  75                  80

Pro Trp Pro His Phe Cys Tyr Tyr Trp Lys Asp Glu Arg Ala Tyr Ala
                85                  90                  95

Phe Arg Glu Ala Leu Ala Ala Leu Leu Phe Thr Ile Gly Lys Ile Arg
            100                 105                 110

Lys Ser Ile Arg Ile Gln Arg Gly Pro Gly Arg Thr Phe Gly Ile Trp
        115                 120                 125

Val Asn
    130



<210> 21
<211> 132
<212> PRT
<213> Artificial Sequence

<220>
<223> Description of Artificial Sequence:Designed
      Peptide

<400> 21
Met Arg Gly Ser His His His His His His Gly Ser Val Asp Gly Thr
  1               5                  10                  15

Pro Lys Asp Ile Arg Ile Gln Arg Gly Pro Gly Arg Thr Phe Val Thr
             20                  25                  30

Ile Gly Glu Arg Ala Tyr Ala Phe Arg Glu Ala Leu Ala Ala Leu Leu
         35                  40                  45

Leu Leu Leu Ala Lys Glu His Thr His Ser Glu Arg Pro Trp Pro His
     50                  55                  60

Phe Cys Tyr Tyr Trp Lys Ala Lys Glu His Thr His Ser Glu Arg Pro
 65                  70                  75                  80

Trp Pro His Phe Cys Tyr Tyr Trp Lys Ala Lys Glu His Thr His Ser
                 85                  90                  95

Glu Arg Pro Trp Pro His Phe Cys Tyr Tyr Trp Arg Lys Asn Ile Arg
            100                 105                 110

Ile Gln Arg Gly Pro Gly Arg Thr Phe Val Thr Ile Gly Gly Gly Ser
        115                 120                 125

Gly Leu Ile Asn
    130



<210> 22
<211> 11
<212> PRT

<213> Artificial Sequence

<220>
<223> Description of Artificial Sequence:Designed
      Peptide

<400> 22
Tyr Gly Arg Lys Lys Arg Arg Glu Arg Arg Arg
 1               5                   10


<210> 23
<211> 34
<212> DNA
<213> Artificial Sequence

<220>
<223> Description of Artificial Sequence:Designed Base
      Sequence

<400> 23
gtacgggcgg aagaagcggc ggcagcggcg gcgc                                34


<210> 24
<211> 11
<212> PRT
<213> Artificial Sequence

<220>
<223> Description of Artificial Sequence:Designed
      Peptide

<400> 24
Val Arg Ala Glu Glu Ala Ala Ala Ala Ala Ala
 1               5                   10


<210> 25
<211> 12
<212> PRT
<213> Artificial Sequence

<220>
<223> Description of Artificial Sequence:Designed
      Peptide

<400> 25
Leu Arg Arg Phe Gly Asp Lys Leu Asn Leu Arg Gln
1               5                   10

<210> 26
<211> 36
<212> DNA
<213> Artificial Sequence

<220>
<223> Description of Artificial Sequence:Designed Base
      Sequence

<400> 26
ctgcggagat tcggcgacaa gctcaacttg cggcag                                36

<210> 27
<211> 11
<212> PRT
<213> Artificial Sequence

<220>
<223> Description of Artificial Sequence:Designed
      Peptide

<400> 27
Ala Glu Ile Arg Arg Gln Ala Gln Leu Ala Ala
1               5                   10

<210> 28
<211> 69
<212> DNA
<213> Artificial Sequence

<220>
<223> Description of Artificial Sequence:Designed Base
      Sequence

<400> 28
gtacgggcgg aagaagcggc ggcagcggcg gcgctgcgga gattcggcga caagctcaac 60
ttgcggcag                                                          69

<210> 29
<211> 23
<212> PRT
<213> Artificial Sequence

<220>
<223> Description of Artificial Sequence:Designed
      Peptide

<400> 29
Val Arg Ala Glu Glu Ala Ala Ala Ala Ala Ala Leu Arg Arg Phe Gly
  1               5                  10                  15

Asp Lys Leu Asn Leu Arg Gln
             20


<210> 30
<211> 22
<212> PRT
<213> Artificial Sequence

<220>
<223> Description of Artificial Sequence:Designed
      Peptide

<400> 30
Tyr Gly Arg Lys Lys Arg Arg Gln Arg Arg Arg Cys Gly Asp Ser Ala
  1               5                  10                  15

Thr Ser Ser Thr Cys Gly
             20


<210> 31
<211> 22
<212> PRT
<213> Artificial Sequence

<220>
<223> Description of Artificial Sequence:Designed
      Peptide

<400> 31
Thr Gly Gly Arg Ser Gly Gly Ser Gly Gly Ala Ala Glu Ile Arg Arg

1               5                   10                  15

Gln Ala Gln Leu Ala Ala
            20


<210> 32
<211> 72
<212> DNA
<213> Artificial Sequence

<220>
<223> Description of Artificial Sequence:Designed Base
      Sequence

<400> 32
cgtatggccg caagaaacgc cgccaacgcc gccgcgctgc ggagattcgg cgacaagctc 60
aacttgcggc ag                                                      72


<210> 33
<211> 24
<212> PRT
<213> Artificial Sequence

<220>
<223> Description of Artificial Sequence:Designed
      Peptide

<400> 33
Arg Met Ala Ala Arg Asn Ala Ala Asn Ala Ala Ala Leu Arg Arg Phe
1               5                   10                  15

Gly Asp Lys Leu Asn Leu Arg Gln
            20


<210> 34
<211> 23
<212> PRT
<213> Artificial Sequence

<220>
<223> Description of Artificial Sequence:Designed
      Peptide

<400> 34
Tyr Gly Arg Lys Lys Arg Arg Gln Arg Arg Arg Ala Ala Glu Ile Arg
1               5                   10                  15

Arg Gln Ala Gln Leu Ala Ala
            20

## Description

### Technical Field

The present invention relates to a multifunctional base sequence having two or more functions in different reading frames of the base sequence, to an artificial gene to which the multifunctional base sequence is bound in such a manner as plural reading frames emerge, and to an artificial protein which is a translation product of the artificial gene or a derivative thereof.

### Background Art

Since the birth of the evolutionary molecular engineering, proteins constituting the root of reaction of life or gene DNAs encoding these proteins have been artificially created in laboratories. This technique has made it possible to produce enzymes and proteins with novel activities that do not exist in nature, or proteins that are largely different in their structures from natural proteins, and hence these are expected to be applied in various ways to the fields of medicine and engineering. In the evolutionary molecular engineering, an operation is performed to select a molecule with an activity of the interest out of a pool of random polymers of amino acids or nucleotides in block units which constitute a protein or a gene encoding the protein. For example, the team of Szostak and others produced a DNA pool with random sequences of 100 base-length using a DNA synthesizer, transcribed the DNA pool to RNA in vitro, prepared the RNA pool with the 10¹³ diversity, selected RNA molecules specifically binding to a specific dye from the RNA pool, and thus they reported that, with what kind of sequence structure, an RNA molecule would satisfy the given function (Nature, 346: 818-822, 1990). The team of Szostak and others have further succeeded with a similar approach in generating an RNA molecule with more complicated activity called ligase activity (Science, 261: 1411-1418, 1993).

On the other hand, there is a hypothesis saying that genes have arisen from the repeated polymerization of short genes (Proc. Natl. Acad. Sci. USA, 80: 3391-3395, 1983). Furthermore, it is thought that polypeptides which are rich in simple repetitive structures easily form a stable secondary structure, and therefore, in the evolutionary molecular engineering where large proteins or genes are targeted, a technique is required to synthesize macromolecules by the repetitive polymerization of short structure units (Nature, 367: 323-324, 1994). Amethod of rolling-circle synthesis has been reported as a method for obtaining repetitive polymers in short DNA units (Proc, Natl, Acad. Sci. USA, 92: 4641-4645, 1995), however, the method has to undergo multiple steps such as a phosphorylation reaction, a linkage reaction, a polymerization reaction, a double-strand-formation reaction or the like, and hence its reaction system is complicated.

As an efficient and simple preparation method of repetitive polymers of microgenes, the present inventor has proposed a method of microgene polymerization (Publication of the Japanese Laid-Open Patent Application No. 1997-322775), wherein DNA polymerase is made to act on oligonucleotides A and B, in which at least a part of their sequences are complementary to each other, for the polymerization reaction to occur.

Further, the followings are known: DNA encoding an amino acid polymer having repetitive units resembling the repetitive units of natural proteins such as a silk protein, elastin or the like (Publication of Japanese Laid-Open Patent Application No.1998-14586); a gene cassette encoding an artificial protein with repeated amino acid sequences (Specification of US patent No.5089406); synthetic repetitive DNA for the production of large polypeptides with repeated sequences of amino acids (Specification of US patent No.5641648); DNA sequences encoding peptides with repetitive units of amino acids (Specification of US patent No.5770697); and a synthetic repetitive DNA for the production of large polypeptides containing repeated sequences of amino acids (Specification of US patent No.5830713). Still further, it is previously reported to design a DNA fragment wherein one of the six reading frames easily forms α-helical structure and the library constructed by the fragment encodes a stable protein at a high frequency, and likewise, to design a DNA fragment wherein one of the six reading frames easily forms β-strand structure (Proc, Natl, Acad. Sci. USA, 94: 3805-3810, 1997).

The subject of the present invention is to provide: a multifunctional base sequence having two or more functions in different reading frames of the base sequence, which is necessary in creating industrially useful artificial proteins; an artificial gene to which the multifunctional base sequence is linked in such a manner as plural reading frames emerge; and an artificial protein which is a translation product of the artificial gene, or a derivative thereof, and the like.

### Disclosure of the Invention

A basic strategy employed for creating functional artificial molecules targeting nucleic acids such as DNA and RNA, or short peptides is to select a molecule having a function of the interest out of a DNA pool (library) with random sequences as a starting point. However, when this system is adopted for generating polymer proteins just as it is, translation termination codons (TAA, TAG, TGA) would emerge at a high frequency in DNA with random sequences. As a result of this, only a library which produces nothing more than short peptides can be obtained. Therefore, such a system is not practical to use for the protein generation. However, according to the foregoing method of a microgene polymerization (Publication of Japanese Laid-Open Patent Application No.1997-322775) of the present inventor, when tandem polymerization occurs while disorderliness is added to a microgene, a resulting polymer possesses a combinatorial property of plural reading frames which yields considerably large diversity. Besides, this method of micorgene polymerization makes it possible to design a microgene for use to avoid in advance the emergence of translation termination codons in any of the reading frames, and therefore this method has proved that the problem of translation termination codons found at a high frequency in DNA with random sequences can be avoided.

A library produced by the method of microgene polymerization has molecular diversity, yet its property as a whole largely depends on the sequence of a microgene which is used in the first place. Therefore, it is possible to specialize the artificial gene library, which is to be produced, to a certain direction depending on the designing manner of a microgene which is used in block units. However, it has also become clear that sufficient utilization of high-speed processors and genomic information is indispensable to design microgenes that are necessary for producing artificial proteins having biological functions such as: a neutralizing antigen protein for the AIDS virus, which has immune activity of enough strength; an artificial protein with activity to activate innate immunity; an artificial protein which induces the release of TNF-α from mononuclear phagocytes and which is stable in blood; an artificial protein skeleton which evades the surveillance of the human immune system; and the like.

Based on the findings described in the above, the present inventor has considered that an artificial gene capable of expressing functional artificial proteins might be created by the method of microgene polymerization, and has made an attempt to design a single microgene by the method of computational science where the microgene has a plurality of biological functions: it has a protein for the AIDS virus neutralizing antigen as its basic biological function; and in addition to this, it encodes a peptide which has a biological function to easily form secondary structure such as its property of easily forming α-helical structure and the like in different reading frames. The present inventor has chosen a protein for neutralizing antigen for the AIDS virus and the potential for forming α-helical structure as the two biological functions because it has been well known that: some antibodies to the region called Loop 3 of the gp120 protein which is present in AIDS virus have the virus-neutralizing activity; an antibody to the Loop 3 is hardly obtained since the Loop 3 region is hidden inside the gp120 protein; and a synthesized peptide for Loop 3 neutralizing antigen has weak immunogenicity so that the antibody would not be obtained as expected, and because an anti-loop antibody, which is to become a candidate for the neutralizing antibody, may possibly be obtained by creating an artificial protein with strong immunogenicity which has Loop 3 peptide sequences at least in some parts and is entirely and stably supported by the α-helix skeleton and by using the artificial protein as an immunogen.

To start with, a microgene is designed which encodes, in one of the reading frames, a peptide "RKSIRIQRGPGRTFVTIGKI" which is known as a neutralizing antigen for AIDS virus. For instance, when a microgene is designed by selecting a specific codon from among six codons, "CGT", "CGC", "CGA", "CGG", "AGA" and "AGG" that correspond to the first R (arginine), then selecting a specific codon from either of two codons, "AAA" and "AAG" that correspond to the next K "lysine", and sequentially selecting specific codons thereafter in a similar way, the variants of base sequences encoding the aforementioned peptide for the neutralizing antigen in one of the reading frames piles up to approximately 1651×10⁸ under the degeneracy of codons. Further, a single microgene has three reading frames each in both plus- and minus-strands, which enabling it to encode six different peptides. For example, two peptides which are totally different from the above-mentioned neutralizing antigen peptide would be encoded in the two different reading frames in the same direction. Therefore, base sequences encoding a peptide having the property of "easily forming secondary structure" in either of the two other reading frames in the same direction were searched using a processor from among about 1651×10⁸ variants of base sequences as described above.

The search was performed with Enterprise 250 of Sun as a processor. As a result, it was found unrealistic to calculate all base sequences of about 1651×10⁸ variants at the same time, thus the calculation was carried out for those peptides comprising 13 amino acids of "IRIQRGPGRTFVT", which was yielded by deleting both ends of the aforementioned neutralizing antigen peptide. All the base sequences with the possibility of encoding this peptide in one reading frame were listed up on the processor, and in consequence, about 5×10⁸ variants of base sequences were produced. The other two reading frames in the same direction of these 5×10⁸ base sequence variants were translated and after excluding those variants in which the translation was terminated by the emergence of a termination codon and excluding the duplications of peptide sequences, a pool of peptide sequences consisting of about 1506×10⁴ variants was produced on the processor. Next, out of the peptides of about 1506×10⁴ variants, those with the property of "easily forming secondary structure" were individually calculated and scored using a secondary structure prediction program. Although the calculation required a period of more than one week, a base sequence, "ATACGCATTCAGAGAGGCCCTGGCCGCACTTTTGTTACT" was successfully selected as a base sequence which very easily forms α-helix in the second reading frame by sorting the obtained results in a high-to-low score order.

The calculation described above was carried out for the 13-amino acid residues located in the middle part of the 20-amino acid residues of the peptide for AIDS virus neutralizing antigen, and therefore, the uncalculated parts of both end were also calculated similarly. Both of the results obtained by these calculations were combined to give birth to the microgene "Design-25". This microgene "Design-25" can be described as follows: it encodes a sequence for a neutralizing antigen in one of its reading frames; it does not have termination codons in the other two reading frames; it further encodes a peptide with a property of easily forming α-helix in either of the two reading frames; it has submerged structures of two biological functions , which are "AIDS virus neutralizing antigenicity" and "potential for structure formation". Next, the microgene "Design-25" was polymerized by utilizing the method of microgene polymerization invented by the present inventor as described earlier (Publication of Japanese Laid-Open Patent Application No.1997-322775) and the artificial gene libraries consisting of various artificial genes that are complex combinations of "sequences for neutralizing antigen" and "sequences that easily form α-helix" were constructed. By utilizing these artificial gene libraries, artificial proteins of various kinds were expressed in E. coli and it was confirmed that an artificial protein can be obtained which has sequences for AIDS virus neutralizing antigen in some parts thereof while being supported, as a whole, with the α-helical structure, and thus the present invention was accomplished.

The present invention relates to: a multifunctional base sequence wherein the base sequence has two or more functions in different reading frames of the base sequence (claim 1); the multifunctional base sequence according to claim 1, wherein the base sequence is a double-stranded base sequence (claim 2); the multifunctional base sequence according to claim 1 or 2, wherein the base sequence is DNA (claim 3); the multifunctional base sequence according to claim 1 or 2, wherein the base sequence is RNA (claim 4); the multifunctional base sequence according to any of claims 1-4, wherein the base sequence is a linear base sequence (claim 5); the multifunctional base sequence according to any of claims 1-5, wherein all three reading frames emerging from the one-by-one frameshifts of reading frames in the base sequence are devoid of termination codons (claim 6); the multifunctional base sequence according to any of claims 2-6, wherein all six reading frames of the base sequence are devoid of termination codons (claim 7); the multifunctional base sequence according to claim 6 or 7, wherein termination codons do not emerge at the junction points arising from the polymerization of the multifunctional base sequence (claim 8); the multifunctional base sequence according to any of claims 1-8, wherein the two or more functions are two or more biological functions (claim 9); the multifunctional base sequence according to claim 9, wherein the biological functions are: function of easily forming secondary structures; antigen function to induce neutralizing antibodies; function to activate immunity; function to promote or suppress cell proliferation; function to specifically recognize cancer cells; protein transduction function; cell-death-inducing function; function to present residues that determine antigens; metal-binding function; coenzyme-binding function; function to activate catalysts; function to activate fluorescence signal; function to bind to a specific receptor and to activate the receptor; function to bind to a specific factor involved in signal transduction and to modulate the action of the factor; function to specifically recognize biopolymers; cell adhesion function; function to localize proteins to the cell exterior; function to target at a specific intracellular organelle; function to be embedded in the cell membrane; function to form amyloid fibers; function to form fibrous proteins; function to form a protein gel; function to form a protein film; function to form a single molecular membrane; self-aggregation function; function to form particles; or function to assist the formation of higher-order structure of other proteins (claim 10); the multifunctional base sequence according to any of claims 1-10, wherein the base sequence comprises 15-500 bases or base pairs (claim 11); the multifunctional base sequence according to any of claims 1-11, wherein the multifunctional base sequence is modified for polymerization (claim 12); and the multifunctional base sequence according to any of claims 1-12, wherein a natural base sequence is linked thereto (claim 13).

The present invention further relates to a method of producing a multifunctional base sequence having two or more functions wherein a base sequence is selected, from among all the combinations of base sequences encoding an amino acid sequence having a given function, which has a function same as or different from the given function in a reading frame different from that of the amino acid sequence having the given function (claim 14); the method of producing a multifunctional base sequence having two or more functions according to claim 14, wherein the base sequence, which has a function same as or different from the given function, is selected by the computational science approach (claim 15); the method of producing a multifunctional base sequence having two or more functions according to claim 15, wherein the computational science approach is an approach to make assessments and selections based on the scores obtained by using a biological function prediction program (claim 16); the method of producing a multifunctional base sequence having two or more functions according to any of claims 14-16, wherein the base sequence is a double-stranded base sequence (claim 17); the method of producing a multifunctional base sequence having two or more functions according to any of claims 14-17, wherein the base sequence is DNA (claim 18); the method of producing a multifunctional base sequence having two or more functions according to any of claims 14-17, wherein the base sequence is RNA (claim 19); the method of producing a multifunctional base sequence having two or more functions according to any of claims 14-19, wherein the base sequence is a linear base sequence (claim 20); the method of producing a multifunctional base sequence having two or more functions according to any of claims 14-20, wherein all three reading frames emerging from the one-by-one frameshifts of the reading frames in the base sequence are devoid of termination codons (claim 21); the method of producing a multifunctional base sequence having two or more functions according to any of claims 17-21, wherein all six reading frames of the base sequence are devoid of termination codons (claim 22); the method of producing a multifunctional base sequence having two or more functions according to claim 21 or 22, wherein termination codons do not emerge at the junction points arising from the polymerization of the multifunctional base sequence (claim 23); the method of producing a multifunctional base sequence having two or more functions according to any of claims 14-23, wherein the two or more functions are two or more biological functions (claim 24); the method of producing a multifunctional base sequence having two or more functions according to claim 24, wherein the biological functions are: function of easily forming secondary structures; antigen function to induce neutralizing antibodies; function to activate immunity; function to promote or suppress cell proliferation; function to specifically recognize cancer cells; protein transduction function; cell-death-inducing function; function to present residues that determine antigens; metal-binding function; coenzyme-binding function; function to activate catalysts; function to activate fluorescence signal; function to bind to a specific receptor and to activate the receptor; function to bind to a specific factor involved in signal transduction and to modulate the action of the factor; function to specifically recognize biopolymers; cell adhesion function; function to localize proteins to the cell exterior; function to target at a specific intracellular organelle; function to be embedded in the cell membrane; function to form amyloid fibers; function to form fibrous proteins; function to form a protein gel; function to form a protein film; function to form a single molecular membrane; self-aggregation function; function to form particles; or function to assist the formation of higher-order structure of other proteins (claim 25); the method of producing a multifunctional base sequence having two or more functions according to any of claims 14-25, wherein the base sequence comprises 15-500 bases or base pairs (claim 26); the method of producing a multifunctional base sequence having two or more functions according to any of claims 14-26, wherein modification is further performed for polymerization of the multifunctional base sequence (claim 27); the method of producing a multifunctional base sequence having two or more functions according to any of claims 14-27, wherein a natural base sequence is further bound thereto (claim 28); and a multifunctional base sequence having two or more functions which can be produced by the method of producing a multifunctional base sequence having two or more functions according to any of claims 14-28 (claim 29).

The present invention still further relates to: an artificial gene to which one or more multifunctional base sequences according to any of claims 1-13 or to claim 29 are bound in such a manner as plural reading frames emerge (claim 30); the artificial gene according to claim 30 which comprises 30-100000 bases or base pairs (claim 31); a method of producing an artificial gene wherein one or more multifunctional base sequences according to any of claims 1-13 or to claim 29 are combinatorially polymerized in such a manner as plural reading frames emerge (claim 32); the method of producing an artificial gene according to claim 32 which comprises: adding a specific DNA sequence [A] at the one end of a double-stranded multifunctional base sequence; adding a specific DNA sequence [B] at the another end of the base sequence; preparing DNA sequences [a] and [b] which are at least partially complementary to DNA sequences [A] and [B] respectively; and performing a ligase reaction for the double-stranded multifunctional base sequence by using a single-stranded DNA sequence to which the DNA sequences [a] and [b] are linked (claim 33); the method of producing an artificial gene according to claim 32, wherein polymerase is made to act on two base sequences, which are comprised of the whole or a part of a multifunctional base sequence and which are at least partially complementary to each other, for the polymerase chain reaction to occur (claim 34); an artificial gene expression vector wherein the artificial gene according to claim 30 or 31 is incorporated in an expression vector (claim 35); the artificial gene expression vector according to claim 35, wherein the artificial gene is bound to a natural gene (claim 36); a cell containing an artificial gene expression vector, wherein the artificial gene expression vector according to claim 35 or 36 is introduced into a host cell (claim 37); the cell containing an artificial gene expression vector according to claim 37, wherein the host cell is E. coli (claim 38); an artificial protein or its derivative obtained as a translation product of the artificial gene according to claim 30 or 31 (claim 39); the artificial protein or its derivative according to claim 39, wherein the translation takes place in a cell-free expression system (claim 40); the artificial protein or its derivative according to claim 39, wherein the translation takes place in a cell expression system (claim 41); the artificial protein or its derivative according to claim 41, wherein the cell is the cell containing an artificial gene expression vector according to claim 37 or 38 (claim 42); the artificial protein or its derivative according to any of claims 39-42, wherein a derivative of the artificial protein is: artificial glycoprotein; artificial phospholipid protein; artificial polyethylene glycol-modified protein; artificial porphyrin-binding protein; or artificial flavin-binding protein (claim 43); a method of producing an artificial protein or its derivative, wherein functional molecules are screened from among the translation products of the artificial gene according to claim 30 or 31 (claim 44); a fusion protein or its derivative wherein the artificial protein or its derivative according to any of claims 39-43 and a marker protein and/or a peptide tag are bound (claim 45); a transgenic non-human animal having the potential for expressing the artificial protein or its derivative according to any of claims 39-43 (claim 46); a transgenic plant having the potential for expressing the artificial protein or its derivative according to any of claims 39-43 (claim 47); a drug for the treatment of various diseases, wherein the artificial protein or its derivative according to any of claims 39-43 is contained as an effective component (claim 48); a drug for the diagnosis of various diseases, wherein the artificial protein or its derivative according to any of claims 39-43 is contained as an effective component (claim 49); an artificial biological tissue wherein the artificial protein or its derivative according to any of claims 39-43 is contained as an effective component (claim 50); and an artificial protein polymer material wherein the artificial protein or its derivative according to any of claims 39-43 is contained as an effective component (claim 51).

### Brief Description of Drawings

Fig. 1 shows an example of a flow chart illustrating the computational operation for automated designing of a microgene.
Fig. 2 shows a microgene comprising a designed double-stranded multifunctional DNA sequence of the present invention, and the amino acid sequence encoded by the microgene.
Fig. 3 shows an example of a designed artificial gene of the present invention.
Fig. 4 shows an example of a designed artificial protein of the present invention.
Fig. 5 shows the result of the SDS polyacrylamide gel electrophoresis for the crude extraction of E. coli which expresses an artificial protein derived from a designed artificial gene of the present invention.
Fig. 6 shows the result of the SDS polyacrylamide gel electrophoresis for the artificial protein purifications derived from a designed artificial gene of the present invention.

### Best Mode of Carrying Out the Invention

There is no specific limitation as to a multifunctional base sequence according to the present invention as long as the base sequence has two or more functions in different reading frames of the base sequence. The specific examples of the base sequence are single- or double-stranded DNA or RNA sequence. Further, these sequences can either take linear or cyclic structure. A sequence with linear structure, however, is preferable because polymerization methods for a linear structured sequence have been established. Furthermore, it is preferable that a multifunctional base sequence of the present invention is devoid of termination codons in all three reading frames with one-by-one frameshifts of the reading frame of the base sequence, and especially for a double-stranded base sequence, it is preferable that all six reading frames in the base sequence are devoid of termination codons. Still further, such base sequence is particularly preferable that a termination codon will not emerge at the junction points (binding points) arising from the polymerization of the multifunctional base sequence.

Functions of the multifunctional base sequence of the present invention may roughly be classified into: functions of translation products of the whole or a part of the base sequence; and functions of whole or part of the base sequence per se. The functions of translation products as mentioned above specifically include: function to easily form secondary structures such as α-helix-formation or the like; antigen function to induce neutralizing antibodies for virus or the like; function to activate immunity (Nature Medicine, 3: 1266-1270, 1997); function to promote or suppress cell proliferation; function to specifically recognize cancer cells; protein transduction function; apoptosis-inducing function; function to present residues that determine antigens; metal-binding function; coenzyme-binding function; function to activate catalysts; function to activate fluorescence signal; function to bind to a specific receptor and to activate the receptor; function to bind to a specific factor involved in signal transduction and to modulate the action of the factor; function to specifically recognize biopolymers such as proteins, DNA, RNA, sugar or the like; cell adhesion function; function to localize proteins to the cell exterior; function to target at a specific intracellular organelle (mitochondrion, chloroplast, ER, etc.); function to be embedded in the cell membrane; function to form amyloid fibers; function to form fibrous proteins; function to form a protein gel; function to form a protein film; function to form a single molecular membrane; self-aggregation function; function to form particles; or function to assist the formation of higher-order structure of other proteins. The term "biological function" used in the present invention means these "a function of the whole or a part of a translation product of a base sequence". As for the functions of the base sequence per se as described above are exemplified by the followings: metal-binding function; coenzyme-binding function; function to activate catalysts; function to bind to a specific receptor and to activate the receptor; function to bind to a specific factor involved in signal transduction and to modulate the action of the factor; function to specifically recognize biopolymers such as proteins, DNA, RNA, sugar or the like; function to stabilize RNA; function to modulate the translation efficiency; function to suppress the expression of a specific gene; and so on.

There is no specific limitation as to a method of producing a multifunctional base sequence having two or more functions according to the present invention as long as a method of producing a multifunctional base sequence is to select a base sequence, from among all the combinations of base sequences encoding an amino acid sequence having a given function, which has a function same as or different from the given function in a reading frame different from that of the amino acid sequence having the given function. However, the aforementioned biologic functions are preferable for a given function, and a biological function different from the given function is preferable in view that it can yield diversity. The above-mentioned amino acid sequence having a given function covers every amino acid sequence having a given function and will not be limited to a single amino acid sequence. For instance, if there are three amino acid sequences having a given function, a multifunctional base sequence will be selected out of all the combinations of base sequences encoding the three amino acid sequences. Other than the known sequences such as, for example, a sequence of the aforementioned neutralizing antigen for AIDS virus or a motif structure such as Glu-Leu-Arg or the like held by the α-chemokine which is a cytokine to leukemia, the following unknown sequences are exemplified as an amino acid sequence having such given function: a sequence arising from deletion, substitution or addition of one or more amino acids in the known sequences and having similar functions to those of the known sequences; a common sequence well preserved among organisms, which is involved in a specific biological function; and a sequence comprising an amino acid sequence avoided by an existing human protein, which has the possibility of evading the surveillance of the human immune system.

The length of a multifunctional base sequence of the present invention will not be limited to a particular length. However, base sequences consisting of 15-500 bases or base pairs, particularly, 15-200 bases or base pairs, and more particularly, 15-100 bases or base pairs are preferable for a stable performance of DNA synthesis. Further, the following multifunctional base sequences may be used as a multifunctional base sequence of the present invention: a multifunctional base sequences which is modified for polymerization by formation of random polymer of microgene (Publication of Japanese Laid-Open Patent Application No.1997-154585) or by the method of microgene polymerization (Publication of Japanese Laid-Open Patent Application No.1997-322775) as described earlier, or by the like; and a multifunctional base sequence to which a natural base sequence is bound.

Base sequences having biological functions that are same as or different from the given functions can be selected by the computational science approach utilizing a computer. These approaches are exemplified more specifically by an approach in which selection is made using scores obtained by a biological function prediction program. Such biological function prediction program is exemplified by a program produced by statistically treating the correlations between biological functions of proteins and peptides and the primary structure of proteins and peptides. The potential for secondary structure formation of a peptide, for instance, can be assessed by utilizing a previously reported protocol (Structure, Function, and Genetics 27: 36-46, 1997). By using this method, the possibility of α-helix- and β-strand-formation predicted at the each residue position of the given peptide sequences is numerically displayed (larger values for higher possibility). The potential levels for α-helix- and β-strand-formation at all the residues of the given peptide sequences are totaled respectively and calculated as a probability of α-helix-formation of the given peptide sequences and a probability of β-strand-formation of the given peptide sequences, and then can be used for the assessment. Other than the above, the following programs are exemplified as function prediction programs: protein family data basis such as "Motiffind program" (Protein Sci., 5: 1991-1999, 1996) and the like for detecting the similarities to known motifs registered to, for example, "PROSITE" (Nucleic Acids Res., 27:215-219, 1999); a similarity searching program "blast" for predicting functions based on the similarities to natural proteins (J. Mol. Biol., 215: 403-410, 1990); "SMART" program for calculating the similarities to various protein factors of the signal transduction system (Proc. Natl. Acad. Sci. USA, 95: 5857-5864, 1998); "PSORT" program for assessing the potential to localize proteins to the cell exterior or to intracellular organelles (Biochem. Sci., 24: 34-35, 1999); "SOSUI" program for assessing the potential to be embedded in the cell membrane (Bioinformatics, 4: 378-379, 1998); and so on.

Sequences obtained by binding two or more multifunctional base sequences of the different kind with ligase or the like, or by binding a multifunctional base sequence to a natural base sequence with ligase or the like can be adopted as a multifunctional base sequence of the present invention. Further, a sequence obtained by separately producing the parts of the multifunctional sequence of the present invention and then binding these parts with ligase or the like can also be adopted as a multifunctional base sequence of the present invention. Still further, a sequence having two or more functions produced by the method of producing a multifunctional base sequence of the present invention as described above is also included in the multifunctional base sequence of the present invention.

The artificial gene of the present invention can be produced by polymerizing one or more variants of the above-mentioned multifunctional base sequences in a combinatorial manner so that a plurality of reading frames emerge. As a method of combinatorial polymerization wherein plural reading frames emerge, methods developed by the present inventor described in the Publication of Japanese Laid-Open Patent Application No.1997-154585 and in the Publication of Japanese Laid-Open Patent Application No.1997-322775 are specifically exemplified. In other words, the former is the method of random polymerization of microgenes using a plurality of microgenes which comprises the following steps: specific DNA sequences different from each other are added to the both ends of a double-stranded multifunctional base sequence; a DNA sequence is prepared which contains at least a part of sequences complementary to these specific DNA sequences, respectively; to perform the ligase reaction of the multifunctional base sequence as aforementioned by using a single-stranded DNA in which the DNA sequences prepared respectively are connected. And the latter is a method of microgene polymerization where a single microgene is repeatedly duplicated and wherein DNA polymerase is made to act on oligonucleotides A and B in which at least a part of their sequences are complementary to each other, to carry out polymerization chain reaction.

There is no limitation as to an artificial gene expression vector of the present invention as long as the expression vector is capable of expressing an artificial gene incorporated therein in the cell of the interest or the like. The specific examples of such expression vectors include: pKC30; pTrc99A; pBluescript II; pSV2-neo; pCAGGS; pcDL-SR α 296; pG-1; pAc373; pQE-9; pET-3a; and the like. If necessary, replication origins, selection markers or promoters, and RNA splicing sites, polyadenylation signals or the like may be added to the above-mentioned vectors. The examples of the aforementioned replication origins include those derived from: SV40; adenovirus; a bovine papillomavirus; ColE1; R factor; F factor; ARS1; and the like. The examples of the promoters include: promoters derived from virus such as retrovirus, polyomavirus, adenovirus, SV40 and the like; the EF1-α promoter derived from chromosome; promoters derived from Bacterio-phage λ; and promoters such as trp, lpp, lac, tac and the like. Further, any selection marker gene may be used as long as a selection marker gene can screen cells containing an artificial gene expression vector of the present invention and the specific examples include: neomycin-resistance gene; puromycin-resistance gene; hygromycin-resistance gene; diphtheria toxin-resistance gene; a fusion gene of β-gal and neo^{R} (β-geo); kanamycin-resistance gene; ampicillin-resistance gene; tetracycline-resistance gene; or the like.

Furthermore, an artificial gene to which a natural gene, such as a gene encoding a peptide having a biological activity or a gene encoding labeling substances such as GFP or the like, is bound at an appropriate location, for example in the upstream, downstream or middle regions with appropriate proportion may also be used as the foregoing artificial gene. As a natural gene for the above case, the one which is incorporated in advance into an expression vector to be used may also be adopted.

A cell containing an artificial gene expression vector of the present invention will not be specifically limited to any host cell as long as the host cell is introduced with the above-mentioned artificial gene expression vector. The examples of such host cells include animal cells, plant cells and microbe cells. These animal cells include insect cells such as Drosophila S2, Spodoptera Sf9 or the like, L cell, CHO cell, COS cell, HeLa cell, C127 cell, BALB/c3T3 cell (including mutants deficient in dihydrofolate reductase, tyhmidine kinase, etc.), BHK21 cell, HEK293 cell, Bowes melanoma cell and the like. Plant cells are exemplified by plant cell strains or the like established from Arabidopsis, Tobacco, Maize, wheat, rice, carrot, soybean and the like. Microbe cells are exemplified by bacterial procaryotic cells such as E. coli, actinomycetes, Bacillus subtilis, Streptococcus, Staphylococcus and the like, and by fungal cells such as Yeast, Aspergillus and the like. Among these, E. coli is preferable because it is rich in its material sources, is well known, and it has suppressor mutants (supD, supE, supF) that translate an amber codon (TAG), which is one of the termination codons, to serine, glutamine or tyrosine.

The above-described artificial gene expression vectors can be introduced into host cells in accordance with methods described in many standard laboratory manuals such as those of Davis and others (BASIC METHODS IN MOLECULAR BIOLOGY, 1986), Sambrook and others (MOLECULAR CLONING: A LABORATORY MANUAL, 2nd Ed., Cold Spring Harbor Laboratory Press, Cold Spring Harbor, N.Y., 1989) and the like, including for instance, calcium-phosphate transfection, DEAE-dextran-mediated transfection, transvection, microinjection, cationic lipid-mediated transfection, electroporation, transduction, scrape loading, ballistic introduction, infection or the like.

There is no particular limitation as to an artificial protein or its derivative of the present invention as long as they are translation products of the aforementioned artificial genes. The artificial genes can be translated by cell-free expression systems or cell expression systems. The cell-free expression systems noted above are exemplified by a cell-free protein synthesis system using the extract of E. coli S30, a cell-free protein synthesis system using wheat embryo and the like. Besides, cell expression systems can be carried out by culturing the cells which contain artificial gene expression vectors mentioned above. Furthermore, examples of the aforementioned derivatives of the artificial proteins include glycoprotein, phospholipid protein, polyethylene-glycol-modified protein, porphyrin-binding protein or flavin-binding protein.

There is no limitation as to a fusion protein or its derivative of the present invention provided that an artificial protein or its derivative and a marker protein and/or a peptide tag are bound, where any conventionally known marker protein may be used. Specific examples of marker proteins are alkaline phosphatase, Fc region of an antibody, HRP, GFP or the like, whereas as for peptide tags, conventionally known peptide tags are specifically exemplified such as Myc tag, His tag, FLAG tag, GST tag and the like. These fusion proteins or their derivatives can be generated according to the usual protocols and are useful for: purification of artificial proteins or the like by utilizing the affinity between Ni-NTA and His tag; detection of artificial proteins; quantification of ligands to artificial proteins or the like. They are also useful as: diagnostic markers for diseases concerning artificial proteins; or reagents for the research in the field. Still further, artificial genes, DNA encoding the artificial proteins, artificial proteins, fusion proteins in which an artificial protein and a marker protein and/or a peptide tag are bound, artificial gene expression vectors, cells which contain artificial gene expression vectors and the like are useful for drugs for the treatment of diseases of various kinds.

A transgenic non-human animal according to the present invention will not be limited to any particular animal as long as it is a non-human animal with the potential for expressing an artificial protein or its derivative. Besides, a transgenic plant according to the present invention will not be limited to any particular plant as long as it is a plant with the potential for expressing an artificial protein or its derivative. Non-human animals of the present invention are specifically exemplified by rodents such as mice, rats or the like, and transgenic plants of the present invention are specifically exemplified by agricultural products such as rice, wheat, maize, soybean, tobacco, carrot or the like. However, they will not be limited to these examples.

In the following, a method of generating a non-human animal, in which a gene encoding an artificial protein or its derivative is expressed on its chromosome, will be explained with reference to the example of a transgenic mouse of an artificial protein or its derivative. A transgenic mouse of an artificial protein or its derivative is generated, for example, by the following steps: a promoter such as a chicken β-actin, a mouse neurofilament, SV40, etc., and poly A such as a rabbit β-globin, SV40 and the like or introns are fused with DNA encoding an artificial protein or its derivative to construct a transgene; this transgene is microinjected into the pronucleus of a mouse fertilezed egg; the resulting egg cell is then cultured and transplanted to the oviduct of a recipient mouse; the transplanted mouse is fed thereafter; and neonate mice having the aforementioned DNA is selected out of the neonate mice that are given birth. The mice with the DNA can be selected by extracting crude DNA from the tails or the like of the mice and then by performing a dot hybridization method with DNA encoding the introduced artificial protein or its derivative as a probe, a PCR method in which a specific primer is used, or the like. Homozygous non-human animals which are born in accordance with Mendel's laws include expression-types for an artificial protein or its derivative and their wild-type littermates. By using both expression-types and their wild-type littermates of homozygous non-human animals at the same time, comparative experiments can accurately be conducted for individual levels with regard to various diagnoses and the like.

Next, a method of generating the above-mentioned transgenic plant for an artificial protein or its derivative will be explained. Transgenic plants can be obtained, for example, by introducing a plant body expression vector integrated with the artificial gene of the present invention into the cells of plant strains established from Arabidopsis, tobacco, maize, wheat, rice, carrot, soybean or the like, and then by regenerating the gene-introduced cell strain in the plant body. Other than a plant body, a protoplast, a callus and part of a plant body (leaf disk, hypocotyl, etc.) are exemplified as a form of a transgenic plant. Furthermore, although Agrobacterium method is preferable for introducing a vector into a host plant cell, other methods such as a polyethylene glycol method, electroporation, a particle gun method or the like may also be employed (EXPERIMENTAL PROTOCOL FOR MODEL PLANTS, Shujunsha, 1996). Besides, by using seeds, tuberous roots, cuttings, mericlones or the like of the transgenic plants of the present invention, the mass production of the plant body of the interest becomes possible.

As an example, a case of a transgenic rice plant is picked out and will be explained in detail below. A callus is induced from a matured seed. Then the callus is infected with Agrobacterium which has been introduced with cDNA of an artificial protein or its derivative. The callus and Agrobacterium are co-cultured and are transferred to a selection medium and are cultured. About three weeks thereafter, the callus is transferred to a redifferentiation medium and is cultured until it is redifferentiated. After having been naturalized for 4-5 days, the callus is transferred to a pot where the transformant can be regenerated (EXPERIMENTAL PROTOCOL FOR MODEL PLANTS, Shujunsha, 1996). Besides, the regeneration methods for carrot, tobacco or the like can be specifically exemplified by the methods of Prof. Kato and Prof. Shono, respectively (TECHNIQUES OF PLANTS OF PLANT TISSUE CULTURE, Asakura Shoten, 1983). Transgenic plants into which DNA encoding an artificial protein or its derivative is introduced can be selected by extracting crude DNA from the interior of the plant body and then by performing methods such as Northern blotting, dot hybridization using DNA which encodes the introduced artificial protein or its derivative as a probe, PCR using a specific primer or the like.

The artificial protein or its derivative may be contained as an effective component in a drug for treatment or diagnosis for various diseases. The artificial protein or its derivative may be used in such a manner as being present in the cells or on the cell membrane. Cell membranes can be obtained, for instance, according to the method of F. Pietri-Rouxel and others (Eur. J. Biochem., 247: 1174-1179, 1997). Further, an artificial protein or its derivative can be collected from the cell culture and purified by any known methods including ammonium sulfate- or ethanol-precipitation, acid extraction, anion- or cation-exchange chromatography, phosphocellulose chromatography, hydrophobic interaction chromatography, affinity chromatography, hydroxy-apatite chromatography and lectin chromatography, where high-performance liquid chromatography is preferably employed. A column to which a ligand to an artificial protein is bound and, when an ordinary peptide tag is added to the artificial protein of the present invention as mentioned below, a column to which a substance having affinity to the peptide tag is bound are used especially in affinity chromatography to obtain an artificial protein.

The present invention will be explained in more detail in the following with reference to the examples. However, the present invention will not be limited to these examples.

### Example 1

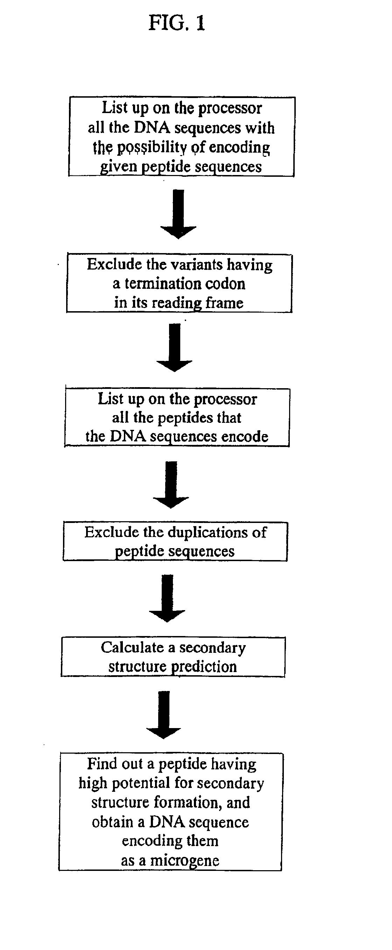

For the purpose of designing a microgene, wherein one of its reading frames makes a base sequence encoding a peptide consisting of an amino acid sequence shown by Seq. ID. No. 1 which is a partial sequence of the gp120 protein belonging to a group of subspecies of the HIV virus, and wherein the microgene can encode a peptide consisting of an amino acid sequence which easily forms secondary structure in at least one of other two reading frames in the same direction, a microgene was constructed according to the flow chart shown in Fig. 1. The throughput of the processor was taken into consideration and the 20-amino acid residues represented by Seq. ID. No. 1 was divided into peptides comprising three amino acid sequences which are partial sequences with partial duplications to each other. In other words, it was divided into peptides comprising three amino acid sequences respectively shown by Seq. ID. Nos. 2, 3 and 4, and the calculation was carried out for each.

For the case of a base sequence encoding a peptide comprising the amino acid sequence shown by Seq. ID. No. 3, about 5×10⁸ base sequences, which were all the base sequences (base length: 13×3=39) capable of encoding a peptide comprising the amino acid sequence shown by Seq. ID. No. 3 in one of the reading frames, were constructed in the processor. Then, among this pool of sequences, approximately 1135×10⁴ base sequences that did not have termination codons in the other two reading frames in the same direction were selected in the processor. Next, all the amino acid sequences, i. e. about 227×10⁵ amino acid sequences, which were encoded by the other two reading frames in the same direction as the reading frames 1 of the base sequences that had been selected were constructed in the processor. From among the peptide pool comprising these amino acid sequences, the duplicating sequences having the same amino acid sequences were excluded and as a result, approximately 1506×10⁴ variant peptide pools each having a different sequence were selected. Each of these peptide pools were assessed for its potential for forming α-helical or β-sheet secondary structure by the scores according to the aforementioned secondary structure prediction program. Then a peptide comprising an amino acid sequence shown by Seq. ID. No. 5 was selected among the peptides that were predicted to possess high potential for forming secondary structure. This peptide was an amino acid sequence encoded by the second reading frame of the base sequence shown by Seq. ID. No. 6 and was predicted to be a sequence which easily forms α-helix.

The calculation was performed in a similar way as above for the base sequence which encodes a peptide comprising the amino acid sequence shown by Seq. ID. No. 2, and a peptide comprising an amino acid sequence shown by Seq. ID. No. 7 was selected which encodes a peptide comprising an amino acid sequence shown by Seq. ID. No. 2 in the first reading frame, and which encodes a peptide with high potential for forming α-helix in the second reading frame, where the amino acid sequence consisting of the position 4-6 amino acid sequences of the peptide is identical to the position 1-3 amino acid sequence within the amino acid sequence shown by Seq. ID. No. 5. This peptide is an amino acid sequence encoded by the second reading frame of the base sequence shown by Seq. ID. No. 8.

A similar calculation as above was performed for Seq. ID. No. 4, and a peptide comprising an amino acid sequence shown by Seq. ID. No. 9 was selected which encodes a peptide comprising an amino acid sequence shown by Seq. ID. No. 4 in the first reading frame, and which encodes a peptide with high potential for forming α-helix in the second reading frame, where the amino acid sequence consisting of the position 1-3 amino acid sequences of the peptide is identical to the position 11-13 amino acid sequence within the amino acid sequence shown by Seq. ID. No. 5. This peptide is an amino acid sequence encoded by the second reading frame of the base sequence shown by Seq. ID. No. 10.

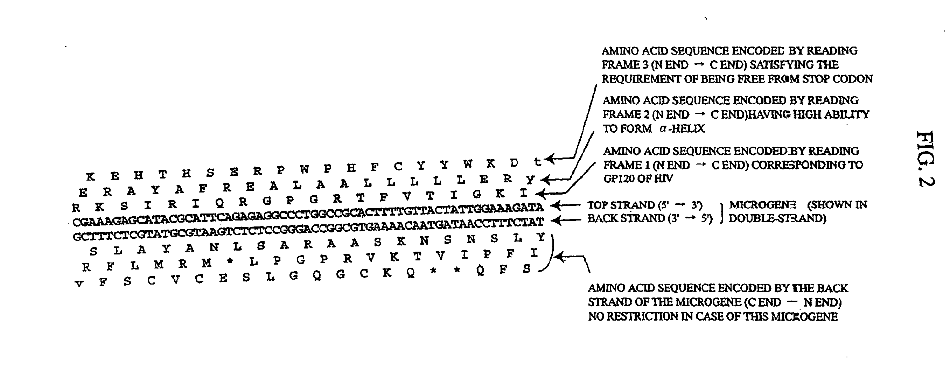

Base sequences obtained by the above-described operations which are respectively represented by Seq. ID. Nos. 6, 8 and 10 were connected while taking their duplications in consideration and the microgene "Design-25" was obtained which has a base sequence shown by Seq. ID. No. 11. As shown in Fig. 2, the designed microgene encodes a partial sequence of the gp120 protein which belongs to a group of subspecies of the HIV virus in the first reading frame and encodes a sequence for a peptide which easily forms α-helical structure in the second reading frame. In the case of the microgene Design-25, no limitation, for instance to avoid the emergence of termination codons, was assigned to the microgene with regard to its minus chain (a complementary sequence to the base sequence shown by Seq. ID. No. 11).

With the above designed microgene "Design-25" as a starting material, a microgene polymer library was constructed by utilizing the technique of the preparation of high-molecular microgene polymer according to the publication of the Japanese Laid-Open Patent Application No. 1997-322775. KY-1197 comprising the base sequence shown by Seq. ID. No. 12 and KY-1198 comprising the base sequence shown by Seq. ID. No. 13 were synthesized to use as oligonucleotide A and oligonucleotide B respectively which were basis for polymerization. 10 residues on the 3' region of oligonucleotide A comprising 34 nucleotides and 10 residues on the 3' region of oligonucleotide B comprising 36 nucleotides were constructed as the sequences complementary to each other except for their 3'-ends.

The conditions for polymerization reaction using the above-described oligonucleotides A and B are as follows at the reaction capacity of 50 µL.

| | |
|---|---|
| KY-1197 | 20 pmol |
| KY-1198 | 20 pmol |
| KCl | 10 mM |
| (NH₄)₂SO₄ | 10 mM |
| Tris-HCl(pH 8.8) | 10 mM |
| MgSO₄ | 2 mM |
| Triton X-100 | 0.1% |
| 2.5 mM dNTP | 7 µL |

The above reaction solution was treated for 10 min at 94.degree. C. and then supplemented with 5.2 units of DNA polymerase (New England Biolabs; "Vent_{R}").

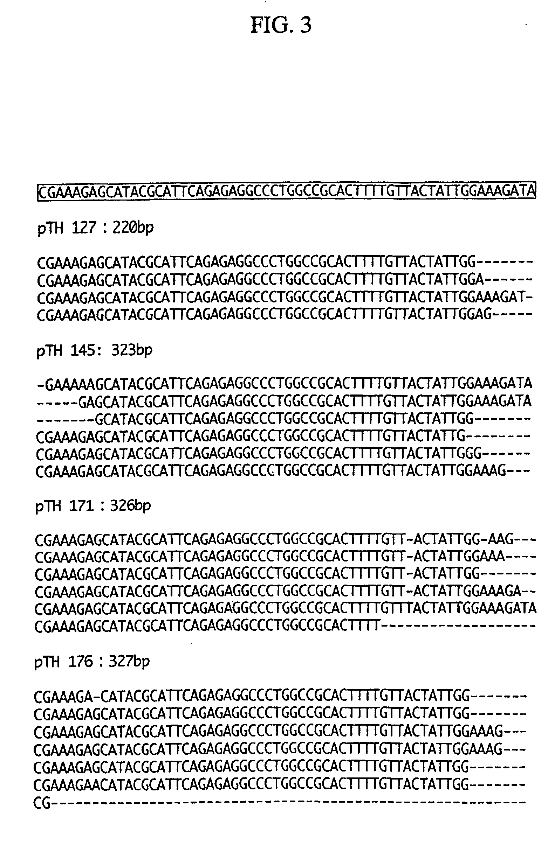

Polymerization reaction was carried out using Perkin-Elmer Gene Amp PCR System 2400. The reaction condition was: performing 55 cycles repeatedly where each cycle consisted of heat-denaturation for 10 sec at 94.degree. C. and annealing and extension reaction for 60 sec at 66.degree. C.; and then performing the final extension reaction for 7 min at 66.degree. C. Artificial genes of the present invention obtained as products of the polymerization reaction were cloned into the plasmid vectors pTZ19R (Protein Eng., 1: 67-74, 1986) and the base sequences of the insertion DNA fragments were determined using a sequencer (Perkin-Elmer). Some of the cloned DNA fragments are shown in Fig. 3. Insertion base sequences of pTH127, pTH145, pTH171 and pTH176 displayed in Fig. 3 are shown by Seq. ID. Nos. 14, 15, 16 and 17, respectively.

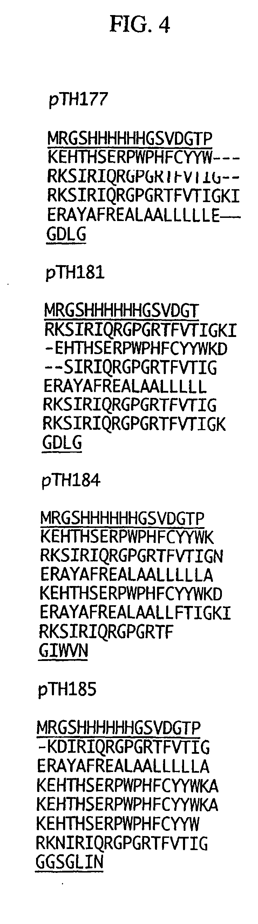

In order to make an artificial gene, the above-mentioned microgene polymer, expressed in E. coli, 24 variants of the insertion DNA fragments cloned into the plasmid vector pTZ19R were excised and re-cloned while taking direction and reading frames in consideration into one of the expression plasmid vector series pKS600-pKS605 which are capable of the direction-and reading-frame-selective expression. These six kinds of expression plasmid vectors pKS600-pKS605 are modifications of the cloning sites of pQE-9, pQE-10 and pQE-11 which are the expression vector series available from Qiagen with the one-by-one frameshifts in reading frames. The expression plasmid vectors pKS600-pKS605 are produced for the purpose of the mass-amount expression of translation products in E. coli, in one of six reading frames which are the total of three each reading frames of plus- and minus-chains. The expression plasmid vectors pTH177-pTH200, to which an artificial gene was inserted, were introduced into E. coli, and the E. coli was then cultured in the presence of IPTG, an expression inducer, and thus an artificial protein having the whole or a part of the amino acid sequence represented by Seq. ID. No. 1 was obtained. Some of the peptide sequences of the translation products are shown in Fig. 4.

Fig. 4 demonstrates that various artificial proteins can be obtained that contain the translation products of plural reading frames of the microgene "Design-25", in a manner that the translation products are admixed in a complicated way. Seq. ID. No. 18 represents the amino acid sequence of the protein produced from pTH177 which was obtained by re-cloning the foregoing artificial gene inserted in pTH127 shown in Fig. 4, into pKS601. In addition, Seq. ID. No. 19 represents the amino acid sequence of the protein produced from pTH181 which was obtained by re-cloning the aforementioned artificial gene inserted in the pTH145, into pKS601. Seq. ID. No. 20 represents the amino acid sequence of the protein produced from pTH184 which was obtained by re-cloning the artificial gene inserted in pTH171, into pKS601. And Seq. ID. No. 21 represents the amino acid sequence of the protein produced from pTH185 which was obtained by re-cloning the artificial gene inserted in pTH176, into pKS601.

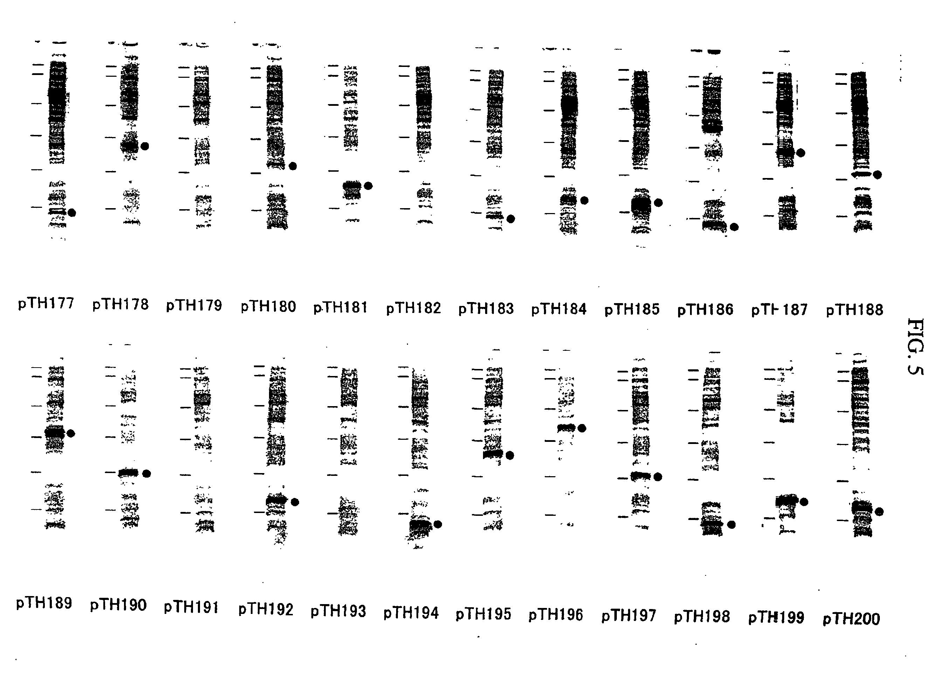

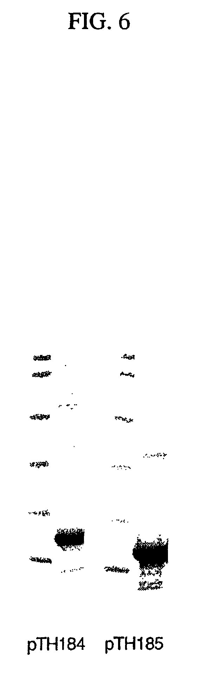

The aforementioned expression vectors pTH177-pTH200, to which 24 kinds of artificial genes were inserted, were introduced into the E. coli strain XLlBlue and induced with IPTG. Then the cell extracts were analyzed by SDS polyacrylamide gel electrophoresis on a 15-25% gradient gel. The results are found in Fig. 5. Molecular mass markers in Fig. 5 are 97400, 66267, 42400, 30000, 20100 and 14400 from the largest, and artificial proteins that are translation products of the artificial genes are indicated with the mark of "●". Further, the expression vectors pKS600-pKS605 are the type of expression vector where a polyhistidine residue is added to its N-terminal, and they can be purified by utilizing a resin which has affinity to this polyhistidine region. Fig. 6 is the results obtained by purifying pTH184 and pTH185 among the artificial proteins observed in Fig. 5, where the purification was carried out using the TALON resin of CLONTECH.

### Example 2

A gene product of HIV called tat has a protein transduction activity; it is introduced in the cell interior upon contact with the cell. Currently, it is reported that the N-terminal sequence of tat is involved in the protein transduction activity and that the amino acid sequence is fused with the N-terminals of various proteins and make the protein introduced into the cell (Science, 285: 1569-1572, 1999). Therefore, with the aforementioned N-terminal amino acid sequence shown by Seq. ID. No. 22 as starting information, a microgene was designed in a similar manner as in Example 1 in accordance with the flow chart illustrated in Fig. 1. The microgene, which has a base sequence shown by Seq. ID. No. 23, encodes the amino acid sequence which has the protein transduction activity and shown by Seq. ID. No. 22, in the second reading frame, and encodes the amino acid sequence which easily forms α-helical structure and shown by Seq. ID. No. 24, in the first reading frame.

On the other hand, it is known that apoptosis is induced when the Noxa protein which has a "BH3" motif, a motif known to be possessed by some proteins of the apoptosis signaling system, is artificially introduced into the cell using such as an adenovirus vector or the like (Science, 288: 1053-1058, 2000). The Noxa protein comprises 100 amino acid residues, has low interspecies conservation in the regions other than the above "BH3" motif, and is a protein rich in α-helix as a whole, and therefore, the above "BH3" motif was used as an amino acid sequence which acts on the apoptosis signaling system and has the apoptosis inducing activity. A microgene was designed in a similar manner as in Example 1 in accordance with the flow chart of Fig. 1, with a "BH3" motif consisting of the amino acid sequence shown by Seq. ID. No. 25 as starting information. This microgene, having the base sequence shown by Seq. ID. No. 26, encodes the amino acid sequence shown by Seq. ID. No. 25, which is thought to have the apoptosis inducing activity, in the first reading frame and encodes the amino acid sequence shown by Seq. ID. No. 27, which easily forms α-helical structure, in the third reading frame.

Next, microgenes respectively having the base sequences shown by Seq. ID. No. 23 described earlier and Seq. ID. No. 26 described in the above were bound to design a microgene "Design-26" which has a base sequence shown by Seq. ID. No. 28. In the first reading frame of the microgene "Design-26" (Seq. ID. No.29), an amino acid sequence which easily forms α-helical structure and a "BH3" motif are fused and the first reading frame is very likely to possess a similar activity to the aforementioned Noxa protein. Furthermore, the second reading frame (Seq. ID. No. 30) contains an amino acid sequence having the protein transduction activity and the third reading frame (Seq. ID. No. 31) contains an amino acid sequence which easily forms α-helical structure.

### Example 3

The microgene "Design-27", which has the protein transduction activity and the apoptosis-inducing activity in different reading frames, was constructed in a similar manner as in Example 2. In this microgene "Design-27" which has the base sequence shown by Seq. ID. No. 32, an amino acid sequence which easily forms α-helical structure and a "BH3" motif are fused in the first reading frame (Seq. ID. No. 33), and the first reading frame is very likely to possess a similar activity to the aforementioned Noxa protein. Further, the third reading frame (Seq. ID. No. 34) consists of a fusion sequence of an amino acid sequence with the protein transduction activity and an amino acid sequence which easily forms α-helical structure.

### Industrial Applicability

Utilization of a multifunctional base sequence having two or more functions in different reading frames of the base sequence or an artificial gene to which the multifunctional base sequence is bound in such a manner as plural reading frames emerge, according to the present invention, makes it possible to find out (generate) industrially useful artificial proteins that do not exist in nature. In addition, the present invention can be applied to the field of materials engineering for producing a stimuli-responsive gel derived from a protein or a nano-scale protein structural body with self-aggregation potential, or the like, and to the field of regenerative medicine for generating an artificial matrix protein which would be the basis of cell proliferation by embedding the motif of an adhesion protein, or the like.

## Claims

1. A multifunctional base sequence wherein the base sequence has two or more functions in different reading frames of the base sequence.

2. The multifunctional base sequence according to claim 1, wherein the base sequence is a double-stranded base sequence.

3. The multifunctional base sequence according to claim 1 or 2, wherein the base sequence is DNA.

4. The multifunctional base sequence according to claim 1 or 2, wherein the base sequence is RNA.

5. The multifunctional base sequence according to any of claims 1-4, wherein the base sequence is a linear base sequence.

6. The multifunctional base sequence according to any of claims 1-5, wherein all three reading frames emerging from the one-by-one frameshifts of reading frames in the base sequence are devoid of termination codons.

7. The multifunctional base sequence according to any of claims 2-6, wherein all six reading frames of the base sequence are devoid of termination codons.

8. The multifunctional base sequence according to claim 6 or 7, wherein termination codons do not emerge at the junction points arising from the polymerization of the multifunctional base sequence.

9. The multifunctional base sequence according to any of claims 1-8, wherein the two or more functions are two or more biological functions.

10. The multifunctional base sequence according to claim 9, wherein the biological functions are: function of easily forming secondary structures; antigen function to induce neutralizing antibodies; function to activate immunity; function to promote or suppress cell proliferation; function to specifically recognize cancer cells; protein transduction function; cell-death-inducing function; function to present residues that determine antigens; metal-binding function; coenzyme-binding function; function to activate catalysts; function to activate fluorescence signal; function to bind to a specific receptor and to activate the receptor; function to bind to a specific factor involved in signal transduction and to modulate the action of the factor; function to specifically recognize biopolymers; cell adhesion function; function to localize proteins to the cell exterior; function to target at a specific intracellular organelle; function to be embedded in the cell membrane; function to form amyloid fibers; function to form fibrous proteins; function to form a protein gel; function to form a protein film; function to form a single molecular membrane; self-aggregation function; function to form particles; or function to assist the formation of higher-order structure of other proteins.

11. The multifunctional base sequence according to any of claims 1-10, wherein the base sequence comprises 15-500 bases or base pairs.

12. The multifunctional base sequence according to any of claims 1-11, wherein the multifunctional base sequence is modified for polymerization.

13. The multifunctional base sequence according to any of claims 1-12, wherein a natural base sequence is linked thereto.

14. A method of producing a multifunctional base sequence having two or more functions wherein a base sequence is selected, from among all the combinations of base sequences encoding an amino acid sequence having a given function, which has a function same as or different from the given function in a reading frame different from that of the amino acid sequence having the given function.

15. The method of producing a multifunctional base sequence having two or more functions according to claim 14, wherein the base sequence, which has a function same as or different from the given function, is selected by the computational science approach.

16. The method of producing a multifunctional base sequence having two or more functions according to claim 15, wherein the computational science approach is an approach to make assessments and selections based on the scores obtained by using a biological function prediction program.

17. The method of producing a multifunctional base sequence having two or more functions according to any of claims 14-16, wherein the base sequence is a double-stranded base sequence.

18. The method of producing a multifunctional base sequence having two or more functions according to any of claims 14-17, wherein the base sequence is DNA.

19. The method of producing a multifunctional base sequence having two or more functions according to any of claims 14-17, wherein the base sequence is RNA.

20. The method of producing a multifunctional base sequence having two or more functions according to any of claims 14-19, wherein the base sequence is a linear base sequence.

21. The method of producing a multifunctional base sequence having two or more functions according to any of claims 14-20, wherein all three reading frames emerging from the one-by-one frameshifts of the reading frames in the base sequence are devoid of termination codons.

22. The method of producing a multifunctional base sequence having two or more functions according to any of claims 17-21, wherein all six reading frames of the base sequence are devoid of termination codons.

23. The method of producing a multifunctional base sequence having two or more functions according to claim 21 or 22, wherein termination codons do not emerge at the junction points arising from the polymerization of the multifunctional base sequence.

24. The method of producing a multifunctional base sequence having two or more functions according to any of claims 14-23, wherein the two or more functions are two or more biological functions.

25. The method of producing a multifunctional base sequence having two or more functions according to claim 24, wherein the biological functions are: function of easily forming secondary structures; antigen function to induce neutralizing antibodies; function to activate immunity; function to promote or suppress cell proliferation; function to specifically recognize cancer cells; protein transduction function; cell-death-inducing function; function to present residues that determine antigens; metal-binding function; coenzyme-binding function; function to activate catalysts; function to activate fluorescence signal; function to bind to a specific receptor and to activate the receptor; function to bind to a specific factor involved in signal transduction and to modulate the action of the factor; function to specifically recognize biopolymers; cell adhesion function; function to localize proteins to the cell exterior; function to target at a specific intracellular organelle; function to be embedded in the cell membrane; function to form amyloid fibers; function to form fibrous proteins; function to form a protein gel; function to form a protein film; function to form a single molecular membrane; self-aggregation function; function to form particles; or function to assist the formation of higher-order structure of other proteins.

26. The method of producing a multifunctional base sequence having two or more functions according to any of claims 14-25. wherein the base sequence comprises 15-500 bases or base pairs.

27. The method of producing a multifunctional base sequence having two or more functions according to any of claims 14-26, wherein modification is further performed for polymerization of the multifunctional base sequence.

28. The method of producing a multifunctional base sequence having two or more functions according to any of claims 14-27, wherein a natural base sequence is further bound thereto.

29. A multifunctional base sequence having two or more functions which can be produced by the method of producing a multifunctional base sequence having two or more functions according to any of claims 14-28.

30. An artificial gene to which one or more multifunctional base sequences according to any of claims 1-13 or to claim 29 are bound in such a manner as plural reading frames emerge.

31. The artificial gene according to claim 30 which comprises 30-100000 bases or base pairs.

32. A method of producing an artificial gene wherein one or more multifunctional base sequences according to any of claims 1-13 or to claim 29 are combinatorially polymerized in such a manner as plural reading frames emerge.

33. The method of producing an artificial gene according to claim 32 which comprises: adding a specific DNA sequence [A] at the one end of a double-stranded multifunctional base sequence; adding a specific DNA sequence [B] at the another end of the base sequence; preparing DNA sequences [a] and [b] which are at least partially complementary to DNA sequences [A] and [B] respectively; and performing a ligase reaction for the double-stranded multifunctional base sequence by using a single-stranded DNA sequence to which the DNA sequences [a] and [b] are linked.

34. The method of producing an artificial gene according to claim 32, wherein polymerase is made to act on two base sequences, which are comprised of the whole or a part of a multifunctional base sequence and which are at least partially complementary to each other, for the polymerase chain reaction to occur.

35. An artificial gene expression vector wherein the artificial gene according to claim 30 or 31 is incorporated in an expression vector.

36. The artificial gene expression vector according to claim 35, wherein the artificial gene is bound to a natural gene.

37. A cell containing an artificial gene expression vector, wherein the artificial gene expression vector according to claim 35 or 36 is introduced into a host cell.

38. The cell containing an artificial gene expression vector according to claim 37, wherein the host cell is E. coli.

39. An artificial protein or its derivative obtained as a translation product of the artificial gene according to claim 30 or 31.

40. The artificial protein or its derivative according to claim 39, wherein the translation takes place in a cell-free expression system.

41. The artificial protein or its derivative according to claim 39, wherein the translation takes place in a cell expression system.

42. The artificial protein or its derivative according to claim 41, wherein the cell is the cell containing an artificial gene expression vector according to claim 37 or 38.

43. The artificial protein or its derivative according to any of claims 39-42, wherein a derivative of the artificial protein is: artificial glycoprotein; artificial phospholipid protein; artificial polyethylene glycol-modified protein; artificial porphyrin-binding protein; or artificial flavin-binding protein.

44. A method of producing an artificial protein or its derivative, wherein functional molecules are screened from among the translation products of the artificial gene according to claim 30 or 31.

45. A fusion protein or its derivative wherein the artificial protein or its derivative according to any of claims 39-43 and a marker protein and/or a peptide tag are bound.

46. A transgenic non-human animal having the potential for expressing the artificial protein or its derivative according to any of claims 39-43.

47. A transgenic plant having the potential for expressing the artificial protein or its derivative according to any of claims 39-43.

48. A drug for the treatment of various diseases, wherein the artificial protein or its derivative according to any of claims 39-43 is contained as an effective component.

49. A drug for the diagnosis of various diseases, wherein the artificial protein or its derivative according to any of claims 39-43 is contained as an effective component.

50. An artificial biological tissue wherein the artificial protein or its derivative according to any of claims 39-43 is contained as an effective component.

51. An artificial protein polymer material wherein the artificial protein or its derivative according to any of claims 39-43 is contained as an effective component.
